# EUROPEAN PATENT SPECIFICATION

(11) **EP 2 452 637 B1**
(45) Date of publication and mention of the grant of the patent: **09.04.2014**
(21) Application number: 10820393.6
(22) Date of filing: 17.09.2010
(51) Int. Cl.: A61B 17/22, A61B 17/00, A61B 17/32, A61M 25/00

(54) **CATHETER WITH MECHANISM FOR REMOVING OBJECT THAT OCCLUDES DUCT OF TUBULAR ORGAN**
KATHETER MIT MECHANISMUS ZUR ENTFERNUNG VON DIE TUBUSLEITUNG VERSTOPFENDEN OBJEKTEN
CATHÉTER AVEC MÉCANISME POUR RETIRER UN OBJET QUI OCCLUT UN CONDUIT D'UN ORGANE TUBULAIRE

(30) Priority: 29.09.2009 JP 2009225299
(43) Date of publication of application: 16.05.2012
(73) Proprietor: Terumo Kabushiki Kaisha, Tokyo 151-0072 (JP)
(72) Inventor: KUMOYAMA, Kenichi, Fujinomiya-shi Shizuoka 418-0015 (JP); KUNIYASU, Junko, Fujinomiya-shi Shizuoka 418-0015 (JP)
(74) Representative: Dossmann, Gérard
(86) International application number: PCT/JP2010/066206
(87) International publication number: WO 2011/040274

(56) References cited:
- WO-A1-2009/036818
- WO-A2-2008/005888
- JP-A- 2004 508 096
- JP-A- 2004 514 463
- JP-A- 2007 236 471
- JP-A- 2007 289 394
- US-A1- 2002 058 956
- US-A1- 2009 031 871
- US-A1- 2009 182 362
- US-B1- 6 482 209
- US-B1- 6 537 279

## Description

### TECHNICAL FIELD

The present invention relates to a catheter provided with a removal mechanism of a tubular-organ occluding object.

### BACKGROUND ART

In a case in which a tubular-organ inside a living body, for example, a blood vessel of four limbs is occluded by a foreign object and the blood flow is disturbed, it becomes a cause for inducing necrosis or intermittent claudication of finger tissues of a hand and a foot, which are caused by ischemia. Therefore, there have been variously proposed catheters provided with removal mechanisms of foreign objects (tubular-organ occluding objects).

For example, in Patent Document 1 for example, there is disclosed a catheter provided with a rotation cutter including a cylinder sleeve shaped body and a thread extending along a portion of the side surface of the aforesaid body. In Patent Document 2, there is disclosed a catheter provided with a cylindrical cutter including a thread extending along a portion of the side surface thereof and a saw blade which is arranged at an end portion and which is formed so as to extend from the center axis to the external side in the radial direction. In Patent Document 3, there is disclosed a catheter provided with a compound cutter assembly including a cutter whose distant diameter is fixed and a cutter assembly whose proximal diameter is adjustable. In Patent Document 4, there is disclosed a catheter provided with a blade edge assembly including a plurality of blade edge blades which present an appearance shape of approximately a round shape when seen from the axis line direction and of an elliptical or a circular truncated cone shape when seen from the lateral direction, and which are arranged radiation-symmetrically with respect to the center longitudinal axis and also, have differential blade edge surfaces.

Document US 2009/182362 A1 discloses a catheter comprising an insertion member which has a lumen elongated in the inside thereof and a removal mechanism for removing an object occluding a tubular-organ inside the living body, wherein the removal mechanism includes a support portion arranged at a distal portion of the insertion member and a plurality of rotation bodies arranged at the support portion and provided with blade edges for excavating the occluding object, and the support portion includes a rotation axis extending to a direction intersecting with the axis of the insertion_member. Document US 2009/031871 A1 discloses an elongate cutting device having a plurality of rotation bodies arranged at a support portion and provided with blade edges whereby the support portion includes rotation axes extending to a direction perpendicular to the axis of the insertion member.

### Prior-art Technical Document

### Patent Documents

Patent Document 1: Japanese unexamined patent publication H11-347040
Patent Document 2: Japanese unexamined patent publication No. 2000-245741
Patent Document 3: Japanese unexamined PCT patent publication No. 2004-514463
Patent Document 4: Japanese unexamined PCT patent publication No. 2006-514577

### DISCLOSURE OF THE INVENTION

### [Problem to be solved by the Invention]

However, the rotation surface of the cutter disclosed in Patent Documents 1 to 4 is approximately perpendicular with respect to the drive shaft and the blade edge of the cutter faces the tubular-organ wall, so that it is easy for the lumen wall to be damaged and also, since there is provided only a single cutter, there was included such a problem that it is not possible for the ground occluding object to be ground finely so as to be discharged easily to the outside of the body.

The present invention was invented in order to solve the problem involved in the prior art technology mentioned above and is addressed to achieve the object of offering a catheter provided with a removal mechanism which excavates a tubular-organ occluding object inside a living body safely and also more efficiently and which can discharge the removed excavated object easily to the outside of the body.

### [Means for Solving the Problem]

One aspect of the present invention for achieving the object mentioned above lies in a catheter as claimed in claim 1 including:
a sheath portion which has a lumen elongated in the inside thereof and which is inserted into a tubular-organ inside a living body;
an insertion member which has a lumen elongated in the inside thereof and which is arranged freely slidably and also rotatably with respect to the lumen of the sheath portion; and
a removal mechanism for removing occluding object of the tubular-organ inside the living body, wherein
the removal mechanism includes a support portion arranged at a distal portion of the insertion member and a plurality of rotation bodies arranged at the support portion and provided with blade edges for excavating the occluding object, and
the support portion includes a rotation axis extending to a direction intersecting with the axis of the insertion member. Further, points at which the extended lines of the rotation axes of the rotation bodies intersecting with the axis of aforesaid insertion member are different respectively, so that the positions at which the blade edges of the rotation bodies excavate the occluding object become different and it becomes possible to realize an excavation of a broad area.

Another aspect of the present invention for achieving the object mentioned above lies in a catheter including:
a hollow drive shaft arranged freely rotatably inside the lumen of the insertion member;
transmission means for converting the rotation of the drive shaft to the rotation of the rotation body.

### [Effect of the Invention]

According to the present invention, a rotation body is arranged at a support portion so as to surround circumference of an axis of an insertion member and has a rotation axis extending to the direction intersecting with the axis of the insertion member.

Consequently, when removing an occluding object of a tubular-organ inside a living body, the blade edge formed on the rotation body excavates the occluding object without being faced to a lumen wall. For this reason, it is possible to reduce damages of the lumen wall during the excavation. Also, when arranging the approximately conically-shaped rotation bodies having tapers toward the distal directions of the rotation bodies adjacently each other, the rotation bodies rotate in the same direction, so that the blade edges of the neighboring rotation bodies move to directions facing mutually and it is possible to add a larger shearing force onto the occluding object along with going to the bottom surface direction between the neighboring rotation bodies, and it is possible to grind the excavated object into smaller narrow slips and to make a situation in which the slips can be discharged easily to the outside of the body.

In other words, it is possible to provide a catheter which can excavate an occluding object of a tubular-organ inside a living body safely and also more efficiently and which includes a removal mechanism capable of discharging the removed excavated object outside the body easily.

If there is provided excavated-piece suction means communicated with the lumen of the insertion member, if there is arranged, in the removal mechanism, with a center opening portion communicated with the lumen of the insertion member and if the rotation body is positioned at the circumference of the center opening portion, the excavated pieces of the tubular-organ occluding object inside the living body are introduced to the center opening portion, so that the collection thereof becomes easy.

If the shape of the blade edge of the rotation body is constituted so as to pull-in the excavated pieces of the occluding object toward the center opening portion along with the rotation of the rotation body, it is possible to pull-in the excavated pieces of the tubular-organ occluding object inside the living body toward the center opening portion efficiently.

If there is provided liquid discharge means for discharging liquid at a distal portion of the sheath portion and if the liquid discharge means includes a flow path constituted by a space which formed between the lumen of the sheath portion and the outer circumference of the insertion member, the liquid discharged at the distal portion of the sheath portion is accompanied by the excavated pieces of the tubular-organ occluding object inside the living body when being pulled-in to the lumen of the insertion member by way of the center opening portion, so that it is possible to collect the excavated pieces easily and also reliably. Also, an independent flow path is not necessary, so that the liquid discharge means can be simplified.

If there is provided a drive shaft arranged freely rotatably inside the lumen of the insertion member and if the removal mechanism includes transmission means for converting the rotation of the drive shaft to the rotation of the rotation body, it is possible to rotate the rotation body having a blade edge singularly (independently) and, for example, it becomes unnecessary to cause the rotation of the rotation body in a state in which the rotation body is compressed onto the tubular-organ occluding object inside the living body.

If the transmission means includes a bevel gear arranged at a distal portion of the drive shaft, it is possible to simplify the structure thereof.

If there is provided excavated-piece suction means communicated with the lumen of the insertion member and the lumen of the drive shaft and if the removal mechanism includes a center opening portion communicated with the lumen of the insertion member, the excavated pieces of the tubular-organ occluding object inside the living body are sucked by way of the lumen of the insertion member and the lumen of the drive shaft, so that it is possible to collect the object excellently.

Still other objects, features and characteristics of the present invention will become clear by referring to preferable exemplified embodiments which are exemplified in the explanations and the attached drawings hereinafter.

### BRIEF DESCRIPTION OF DRAWINGS

FIG. 1 is a side view for explaining a catheter relating to an exemplified embodiment of the present invention;
FIG. 2 is a cross-sectional view for explaining an occluding object removal mechanism and a liquid discharge system shown in FIG. 1;
FIG. 3 is a cross-sectional view for explaining an excavated-piece suction system shown in FIG. 1;
FIG. 4 is an outward appearance view for explaining the occluding object removal mechanism;
FIG. 5 is a perspective view for explaining the occluding object removal mechanism shown in FIG. 4;
FIG. 6 is a plan view for explaining a first cone shown in FIG. 5;
FIG. 7 is a plan view for explaining a second cone shown in FIG. 5;
FIG. 8 is a plan view for explaining a third cone shown in FIG. 5;
FIG. 9 is a cross-sectional view for explaining a transmission device of the occluding object removal mechanism; and
FIG. 10 is a perspective view of the occluding object removal mechanism shown in FIG. 9.

### BEST MODE FOR CARRYING OUT THE INVENTION

Hereinafter, exemplified embodiments of the present invention will be explained while referring to the drawings.

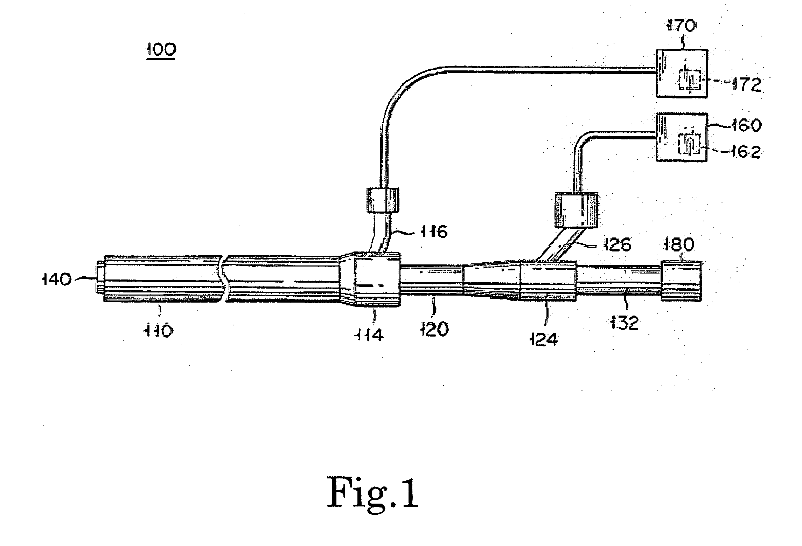

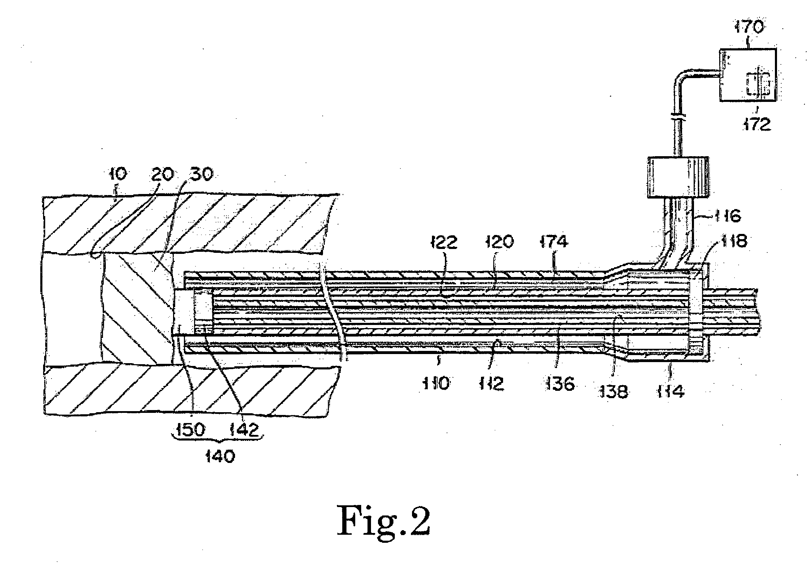

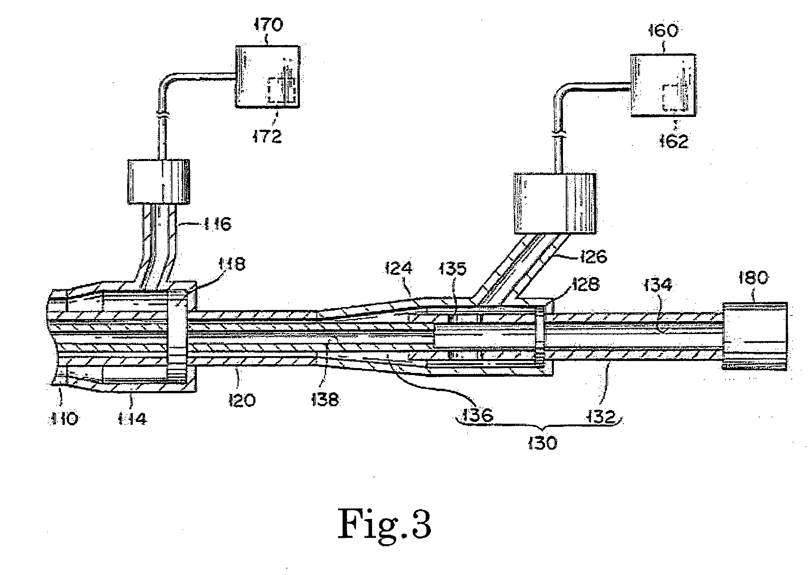

FIG. 1 is a side view for explaining a catheter relating to an exemplified embodiment of the present invention, FIG. 2 is a cross-sectional view for explaining an occluding object removal mechanism and a liquid discharge system shown in FIG. 1, and FIG. 3 is a cross-sectional view for explaining an excavated-piece suction system shown in FIG. 1.

A catheter 100 is used for removing an occluding object of a tubular-organ inside a living body and includes a sheath portion 110, an insertion member 120, a drive shaft 130, an occluding object removal mechanism 140, an excavated-piece suction system 160, a liquid discharge system 170 and a drive device 180.

The tubular-organ inside the living body is, for example, a blood vessel in four limbs of a patient who suffers from atheroma arteriosclerosis, and the occluding object is composed of an atheroma layer which is formed by neutral fat accumulated in an intima layer of a blood vessel and by lime further deposited thereon and besides that, there can also be cited coagulated aggregate (thrombus) caused by adhesion or agglomeration of blood platelets, or caused by coagulation of blood.

The sheath portion 110 is composed of a hollow tube having a lumen 112 which is elongated in the inside thereof and includes a distal portion inserted into a tubular-organ 20 inside a living body 10 and a proximal end portion interlinked to a Y-shaped adaptor 114. The Y-shaped adaptor 114 includes a branch port 116 with which the liquid discharge system 170 is interlinked.

Although there is no limitation in particular, it is preferable for the outer shape of the sheath portion 110 to be cylindrical and it is set appropriately in response to the tubular-organ 20 inserted therein.

It is possible for the material of the sheath portion 110 to apply, for example, a high-polymer material such as: polyolefin, olefin-based elastomer, polyester, soft polyvinyl chloride, polyurethane, urethane-based elastomer, polyamide, polyamide-based elastomer, polytetrafluoroethylene, fluorine-based elastomer, polyimide, ethylene vinyl acetate copolymer and silicone rubber or the like. The polyolefin is, for example, polypropylene or polyethylene. The olefin-based elastomer is, for example, polyethylene elastomer or polypropylene elastomer. The polyamide-based elastomer is, for example, polyamide elastomer.

In case of forming the sheath portion 110 from a high-polymer material, it is possible, for example, to improve rigidity thereof through a compounding process by utilizing a pipe of a superelastic alloy, or an embedded coil or an embedded mesh, which is composed of a metal.

It is preferable for the distal portion of the sheath portion 110 to have a function as an X-ray contrast marker and, for example, it can be formed by using a resin which contains an X-ray contrast material. The X-ray contrast material is, for example, a powder such as of tantalum, tungsten carbide, bismuth oxide, barium sulfate, platinum or an alloy thereof, a cobalt alloy and the like.

The insertion member 120 is composed of a hollow tube having a lumen 122 which is elongated in the inside thereof and includes a distal portion at which the occluding object removal mechanism 140 is arranged and a proximal end portion which is interlinked to a Y-shaped adaptor 124, and is arranged so as to be freely slidable and also rotatable with respect to the lumen 112 of the sheath portion 110. The proximal end portion of the insertion member 120 is extended by passing through the Y-shaped adaptor 114 with which the proximal end portion of the sheath portion 110 is interlinked and, for example, airtightness is secured by a sealing device 118 having an O-ring. The Y-shaped adaptor 124 includes a branch port 126 with which the excavated-piece suction system 160 is interlinked.

For the outer shape of the insertion member 120, there is no limitation in particular if the insertion member is slidable and also rotatable inside the lumen 112 of the sheath portion 110, but it is preferable to be cylindrical.

It is possible for the material of the insertion member 120 to apply, for example, such as a metal and a high-polymer material with relatively high rigidity or such as a material obtained by combining those appropriately and the like. The metal is, for example, a stainless steel, a Ni-Ti alloy, a Cu-Zn alloy, a cobalt alloy or tantalum. The high-polymer material is, for example, polyamide, polyimide, ultra high molecular weight polyethylene, polypropylene or a fluorine resin.

The drive shaft 130 is constituted by interlinking a proximal shaft 132 and a distal shaft 136.

The proximal shaft 132 is composed of a hollow tube having a lumen 134 which is elongated in the inside thereof and it is interlinked to a drive device 180 by being extended by passing through the Y-shaped adaptor 124. With respect to the passing-through portion of the Y-shaped adaptor 124 and the proximal shaft 132, airtightness thereof is secured, for example, by a sealing device 128 including an O-ring. The proximal shaft 132 includes an opening portion 135 at a region which is positioned inside the Y-shaped adaptor 124.

The distal shaft 136 is composed of a hollow tube having a lumen 138 which is elongated in the inside thereof, is arranged freely rotatably in the lumen 122 of the insertion member 120, and is interlinked to a transmission device of the occluding object removal mechanism 140. It should be noted that the distal shaft 136 is interlinked to the proximal shaft 132 in the vicinity of the Y-shaped adaptor 124. There is no limitation in particular for the outer shape of the distal shaft 136 if the shaft is slidable and also rotatable in the inside of the lumen 122 of the insertion member 120.

In consideration of intensity and rigidity required for transmitting the driving force of the drive device 180, it is possible for the material of the proximal shaft 132 and the distal shaft 136 to apply, for example, such as a metal and a high-polymer material with relatively high rigidity or such as a material obtained by combining those appropriately and the like.

The occluding object removal mechanism 140 includes a body 142 which is a support portion arranged at a distal portion of the insertion member 120, a roller bit 150 which is a excavation portion having first to third cones for removing an occluding object 30 of the tubular-organ 20 inside the living body 10, and a transmission mechanism for converting the rotation of the distal shaft 136 to the rotation of the first to third cones.

It is constituted such that the roller bit 150 accompanies the move of the insertion member 120, is projected from the distal end of the sheath portion 110, and contacts with the occluding object 30. The force for excavating the occluding object 30 occurs caused by rolling of the roller bit 150 on the occluding object surface depending on the rotation of the insertion member 120 or occurs caused by transmitting the rotation of the drive shaft 130 (distal shaft 136) to the roller bit 150 through the transmission mechanism. The body 142 includes a center opening portion communicated with the lumen 122 of the insertion member 120.

The excavated-piece suction system 160 is excavated-piece suction means used for collecting excavated pieces of the occluding object 30 which was removed by the occluding object removal mechanism 140. The excavated-piece suction system 160 includes a suction pump 162 for sucking the excavated pieces of the occluding object 30, is interlinked to the branch port 126 of the Y-shaped adaptor 124 which is interlinked to the proximal end portion of the insertion member 120, and is communicate with the lumen 122 of the insertion member 120. The suction pump 162 is excavated-piece suction means composed of a metering pump such as, for example, a diaphragm pump, a squeeze pump (tube pump), a piston pump, a plunger pump and the like.

The region of the proximal shaft 132 which is positioned inside the Y-shaped adaptor 124 includes the opening portion 135, so that the excavated-piece suction system 160 is communicate with the lumen 134 of the proximal shaft 132, and the distal shaft 136 with which the proximal shaft 132 is interlinked passes-through the lumen 122 of the insertion member 120, reaches the occluding object removal mechanism 140 and is communicate with the outside.

In other words, it is possible for the excavated-piece suction system 160 to utilize both sides of the lumen 122 of the insertion member 120 and the lumens 134, 138 of the drive shaft 130 (proximal shaft 132 and distal shaft 136) and it is possible to collect the excavated pieces of the occluding object 30 excellently. If required, it is also possible to form the drive shaft 130 (proximal shaft 132 and distal shaft 136) to be a solid-core shaft. In this case, it happens that the excavated-piece suction system 160 utilizes only the lumen 122 of the insertion member 120 for the suction (collection).

The liquid discharge system 170 is liquid discharge means used for discharging liquid to the distal portion of the sheath portion. The liquid is, for example, a physiological salt solution. If required, it is also possible to add a thrombolytic agent to the physiological salt solution. The thrombolytic agent is, for example, urokinase or tissue plasminogen activator (t-PA).

The liquid discharge system 170 is interlinked to the branch port 116 of the Y-shaped adaptor 114 to which the proximal end portion of the sheath portion 110 is interlinked and is communicated with the lumen 112 of the sheath portion 110, and there are included a discharge pump 172 interlinked to a container in which liquid is maintained and a flow path 174 for discharging the liquid to the distal portion of the sheath portion 110. The discharge pump 172 is a metering pump such as, for example, a diaphragm pump, a squeeze pump (tube pump), a piston pump, a plunger pump and the like.

The flow path 174 is constituted by a space which is formed between the lumen 112 of the sheath portion 110 and the outer circumference of the insertion member 120. Thus, the liquid discharged to the distal portion of the sheath portion 110 is accompanied by the excavated pieces of the occluding object 30 of the tubular-organ 20 inside the living body 10 when being pulled-in to the lumen 122 of the insertion member 120 by way of the center opening portion of the body 142 of the occluding object removal mechanism 140, so that it is possible to collect the excavated pieces easily and also reliably. In addition, there is not required an independent flow path, so that the liquid discharge system 170 can be simplified.

The drive device 180 includes, for example, a servo motor and it is constituted for the proximal shaft 132 to be driven freely rotatably. The proximal shaft 132 is interlinked to the first to third cones by way of the distal shaft 136 and the transmission mechanism. Consequently, it is possible for the drive device 180 to rotate the first to third cones separately (independently) and to remove the occluding object 30 of the tubular-organ 20 inside the living body 10. If required, it is also possible for the drive device 180 to be eliminated and to rotate the drive shaft 130 (proximal shaft 132) manually.

Next, it will be described in detail with respect to a first exemplified embodiment of the occluding object removal mechanism.

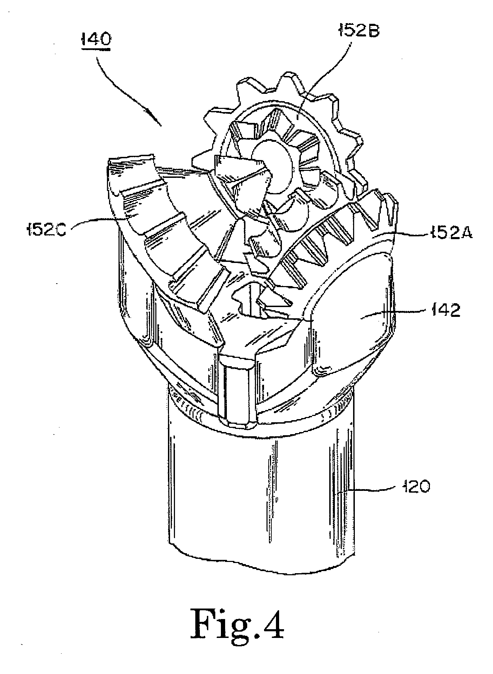

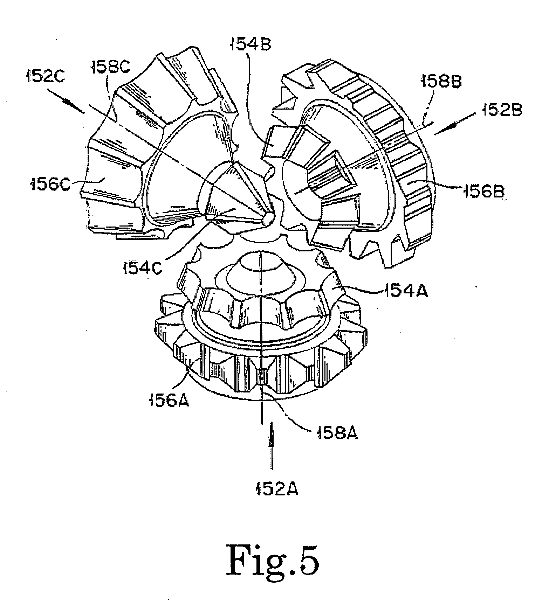

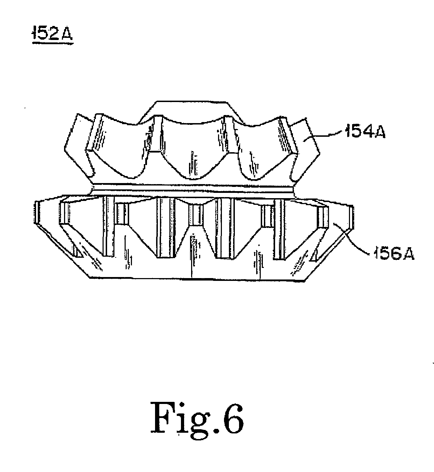

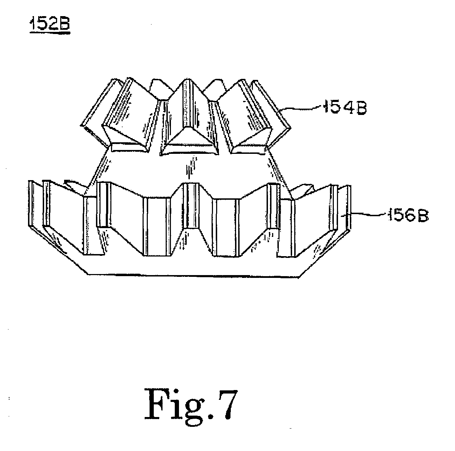

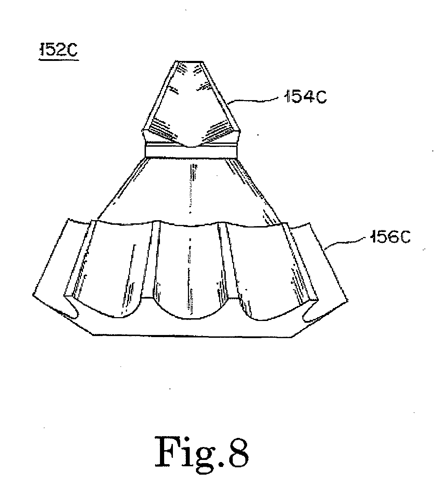

FIG. 4 is an outward appearance view for explaining an occluding object removal mechanism, FIG. 5 is a perspective view for explaining the occluding object removal mechanism shown in FIG. 4, and FIGS. 6 to 8 are plan views for explaining first to third cones shown in FIG. 5.

The occluding object removal mechanism includes a body 142 arranged at the distal portion of the insertion member 120 and first to third cones 152A to 152C which are rotation bodies.

The first to third cones 152A to 152C are arranged, for example, at the body 142 freely rotatably through bearings and are position-set so as to surround the circumference of an axis A of the insertion member 120, and they include upper-portion blade edges 154A to 154C which are arranged at the outer circumferences of the top portions thereof, lower-portion blade edges 156A to 156C which are arranged at the outer circumferences of the proximal portions thereof and rotation axes which are extended to the directions intersecting with the axis A of the insertion member 120. The bearing is formed, for example, by combining a roller bearing and a ball bearing.

Consequently, when removing the occluding object of the tubular-organ inside the living body, the blade edges formed on the rotation bodies excavate the occluding object without facing the lumen wall. Thus, it is possible to reduce damages of the lumen wall during the excavation.

Also, when arranging the rotation bodies adjacently each other, the rotation bodies rotate in the same direction, so that the blade edges of the neighboring rotation bodies move to directions facing mutually and it is possible to add a larger shearing force onto the occluding object between the neighboring rotation bodies, and it is possible to grind the excavated object into smaller narrow slips and to make a situation in which the slips can be discharged easily to the outside of the body. Further, points at which the extended lines of the rotation axes of the rotation bodies intersect with the axis of the insertion member are different respectively, so that the positions at which the blade edges of the rotation bodies excavate the occluding object become different and it becomes possible to realize an excavation of a broad area.

In other words, it is possible to excavate an occluding object of a tubular-organ inside a living body safely and also more efficiently, and to discharge the removed excavated object easily to the outside of the body.

The excavated pieces of the occluding object are ground by the upper-portion blade edges 154A to 154C and the lower-portion blade edges 156A to 156C and become narrower slips, and they are introduced to a center opening portion 144 of the body 142 surrounding the first to third cones 152A to 152C. Consequently, it is possible to repress plugging of the center opening portion 144.

The first to third cones 152A to 152C are formed approximately in conically-shapes having tapers toward the rotation axes 158A to 158C and it is possible for the upper-portion blade edges 154A to 154C and the lower-portion blade edges 156A to 156C to be bitingly-engaged with the occluding object excellently. Also, the upper-portion blade edges 154A to 154C and the lower-portion blade edges 156A to 156C are formed in gear shapes and are arranged by being formed in trains at the outer circumferences of the top portions and the proximal portions of the first to third cones 152A to 152C, in which there is improved biting-engagement property with respect to the occluding object. It should be noted that it is possible for the upper-portion blade edges 154A to 154C and the lower-portion blade edges 156A to 156C to be formed, for example, by mechanically processing the outer circumferences of the first to third cones 152A to 152C or by being embedded thereinto.

Here, it will be described in detail with respect to the first to third cones 152A to 152C.

The blade trains of the upper-portion blade edges 154A to 154C and the lower-portion blade edges 156A to 156C in the first to third cones 152A to 152C are arranged adjacently mutually with the neighboring blade trains of the first to third cones 152A to 152C. Therefore, it becomes possible to hit the upper-portion blade edges 154A to 154C and the lower-portion blade edges 156A to 156C thoroughly from stem to stern without space with respect to the occluding object of the tubular-organ inside the living body.

Also, the shapes of the first to third cones 152A to 152C are not identical cone shapes and each blade train has a different cone angle so as to exert actions of blow and also compression and concurrently, actions of dragging and also scooping. In other words, the shapes of the upper-portion blade edges 154A to 154C and the lower-portion blade edges 156A to 156C are constituted so as to pull-in the excavated pieces of the occluding object of the tubular-organ inside the living body to the center opening portion along with the rotation of the first to third cones 152A to 152C, and it is possible to pull-in (collection) the excavated pieces efficiently.

In this exemplified embodiment, the first to third cones 152A to 152C of the occluding object removal mechanism are arranged at the body 142 freely rotatably and also there is provided the drive device for driving the insertion member 120. The rotation of the first to third cones 152A to 152C is set such that the first to third cones 152A to 152C are rolled on the surface of the occluding object by rotating the insertion member 120 while compressing the first to third cones 152A to 152C onto the occluding object of the tubular-organ inside the living body and by rotating the body 142 which is arranged at the distal portion of the insertion member 120, and the occluding object is to be cut by the lower-portion blade edges 156A to 156C and the upper-portion blade edges 154A to 154C.

In this case, it becomes unnecessary to provide the transmission device for rotationally driving the first to third cones 152A to 152C and the drive shaft (proximal shaft and distal shaft) which is interlinked to the transmission device, so that it is possible to simplify the apparatus. Also, there does not exist an interfering object (transmission device and drive shaft) in the vicinity of the center opening portion 144 of the body 142, so that it is possible to collect the excavated pieces of the occluding object of the lumen inside the living body excellently. It should be noted that the lower-portion blade edges 156A to 156C of the first to third cones 152A to 152C are set to have shapes which cause the rotation easily when rotating the body 142 in a state of compressing the first to third cones 152A to 152C onto the occluding object of the tubular-organ inside the living body.

As mentioned above, in this exemplified embodiment, the first to third cones are arranged at the body so as to surround the circumference of the axis of the insertion member, and include the rotation axes extending in the directions intersecting with the axis of the insertion member, so that the rotation directions of the blade edges arranged at the first to third cones are identical and also, the top surfaces of the first to third cones are adjacent mutually. Consequently, when removing the occluding object of the tubular-organ inside the living body, it becomes in a state in which the blade edges arranged at the first to third cones will excavate the occluding object while bitingly-engaging with the occluding object, so that it is possible to achieve stable removal of the occluding object. In other words, it is possible to provide a catheter provided with a removal mechanism which can stably remove the occluding object of the lumen inside the living body.

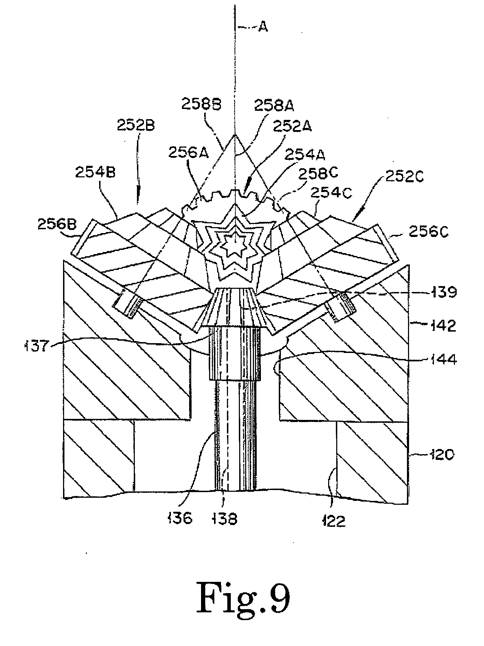

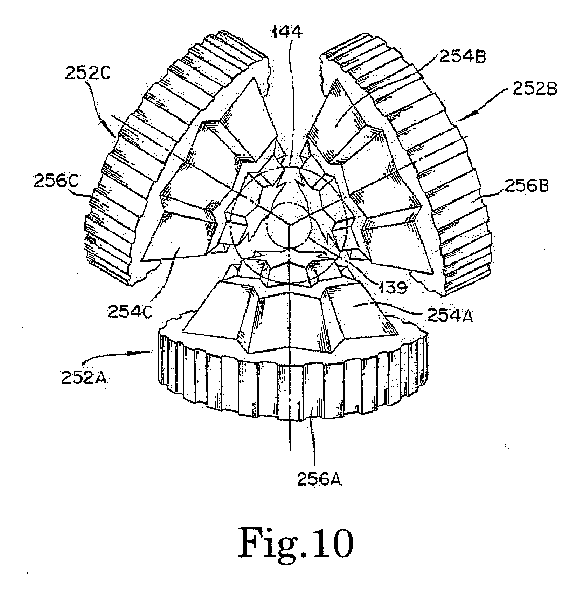

Next, it will be described in detail with respect to a second exemplified embodiment of the occluding object removal mechanism. FIG. 9 is a cross-sectional view for explaining a transmission device of an occluding object removal mechanism and FIG. 10 is a perspective view of the occluding object removal mechanism shown in FIG. 9.

In this exemplified embodiment, there is provided a drive shaft 130 which is arranged freely rotatably inside the lumen 122 of the insertion member 120 and the occluding object removal mechanism includes the transmission device for converting the rotation of the drive shaft 130 to the rotation of first to third cones 252A to 252C, and there is fixed a bevel gear 137 at the distal portion of the drive shaft 130 (distal shaft 136). The bevel gear 137 is constituted so as to be bitingly-engaged with gears 256A to 256C respectively, which have blade edges and which are provided at the lower portions of the first to third cones 252A to 252C, and it is possible to make the transmission device be a simple structure. The bevel gear 137 includes a through-hole 139 which is communicated with the lumen 138 of the distal shaft 136 and is set so as not to interfere with the collection of the excavated pieces, which utilizes the lumen 138 of distal shaft 136.

The first to third cones 252A to 252C are arranged, for example, at the body 142 freely rotatably through bearings and are position-set so as to surround the circumference of an axis A of the insertion member 120, and the first to third cones 252A to 252C include upper-portion blade edges 254A to 254C which are arranged at the outer circumferences of the top portions thereof, lower-portion blade edges 256A to 256C which are arranged at the outer circumferences of the proximal portions thereof and rotation axes 258A to 258C which are extended to the directions intersecting with the axis A of the insertion member 120. The bearing is formed, for example, by combining a roller bearing and a ball bearing.

Consequently, this exemplified embodiment exerts a similar effect as that of the first exemplified embodiment and concurrently, it is possible to directly drive the first to third cones 252A to 252C by the drive device 180, so that even for a soft occluding object in which it is difficult for the cones to rotate only by rotating the insertion member 120 in a state of compressing the cones thereon, it is possible to achieve the excavation safely and also efficiently, and it is possible to discharge the removed excavated object easily to the outside of the body.

The present invention is not limited by the above-mentioned exemplified embodiments and it is possible to be modified variously within the scope of the claims. For example, the tubular-organ inside the living body is not limited by the blood vessel and it is also possible to be applied to a biliary tract for transporting bile to an intestinal tract. In this case, the occluding object of the biliary tract is a gallstone (calculus) composed of, for example, a cholesterol calculus (pure cholesterol stone, combination stone, cholesterol pigment lime stone or mixed stone). Also, the occluding object removal mechanism is not limited by the configuration including three cones and it is also possible to include two cones, or four or more cones appropriately.

### DESCRIPTION OF REFERENCE NUMERALS

10: living body,
20: tubular-organ,
30: occluding object,
100: catheter,
110: sheath portion,
112: lumen,
114: Y-shaped adaptor,
116: branch port,
118: sealing device,
120: insertion member,
122: lumen,
124: Y-shaped adaptor,
126: branch port,
128: sealing device,
130: drive shaft,
132: proximal shaft,
134: lumen,
135: opening portion,
136: distal shaft,
137: bevel gear,
138: lumen,
139: through-hole,
140: occluding object removal mechanism,
142: body,
144: center opening portion,
150: roller bit,
152A to 152C: the first to third cones,
154A to 154C: upper-portion blade edge,
156A to 156C: lower-portion blade edge,
158A to 158C: rotation axis,
160: excavated-piece suction system,
162: suction pump,
170: liquid discharge system,
172: discharge pump,
174: flow path,
180: drive device,
252A to 252C: first to third cones,
254A to 254C: upper-portion blade edge,
256A to 256C: lower-portion blade edge,
258A to 258C: rotation axis.

## Claims

1. A catheter (100) comprising:
a sheath portion (110) which has a lumen (112) elongated in the inside thereof and which is insertable into a tubular-organ (20) inside a living body (10);
an insertion member (120) which has a lumen (122) elongated in the inside thereof and which is arranged freely slidably and rotatably with respect to the lumen (112) of the sheath portion (110); and
a removal mechanism (140) for removing an object (30) occluding the tubular-organ (20) inside the living body (10), wherein
the removal mechanism (140) includes a support portion (142) arranged at a distal portion of the insertion member (120) and a plurality of rotation bodies (152A-152C) arranged at the support portion (142) and provided with blade edges (154A- 154C, 156A-156C) for excavating the occluding object (30), and
the support portion (142) includes a rotation axis extending to a direction intersecting with the axis (A) of the insertion member (120) wherein the support portion (142) includes said plurality of rotation bodies provided with said blade edges for excavating the occluding object, and points at which the extended lines of the rotation axes of the rotation bodies intersect with the axis of the insertion member are different, respectively.

2. The catheter (100) according to claim 1, wherein each rotation body (152A-152C) is approximately in a conical-shape having a taper toward the distal direction of its rotation axis.

3. The catheter (100) according to claim 2, wherein the blade edge of each rotation body is gear shaped and is shaped by forming a train, and the blade train of each rotation body (152A-152C) is arranged so as to rotate mutually independently with respect to a blade train of a neighboring rotation body.

4. The catheter (100) according to any one of claims 1 to 3, further comprising excavated-piece suction means (160, 162) communicated with the lumen (112) of the insertion member (120),
the removal mechanism (140) includes a center opening portion (144) communicated with the lumen of the insertion member (120), and
the rotation bodies are positioned at the circumference of the center opening portion (144).

5. The catheter (100) according to claim 4, wherein a shape of the blade edge of each rotation body is constituted so as to pull-in excavated pieces of the occluding object (30) toward the center opening portion (144) along with the rotation of the rotation bodies.

6. The catheter (100) according to claim 4 or claim 5, further comprising liquid discharge means (170, 172) for discharging liquid at a distal portion of the sheath portion (110), and
the liquid discharge means (170, 172) includes a flow path (174) constituted by a space which is formed between the lumen (112) of the sheath portion (110) and the outer circumference of the insertion member (120).

7. The catheter (100) according to any one of claims 1 to 6, wherein when the removal mechanism (140) is compressed onto the occluding object (30) by projecting the insertion member (120) from the sheath portion (110) and further, when rotating the insertion member (120) at the hand-side, the rotation bodies of the removal mechanism (140) rotate and excavate the occluding object (30), and the excavated object is fed-in inside the lumen (112) of the insertion member (120).

8. A catheter (100) according to claim 1, comprising:
a hollow drive shaft (130) arranged freely rotatably inside the lumen (112) of the insertion member (120); and
transmission means for converting the rotation of the drive shaft (130) to the rotation of the rotation body.

9. The catheter (100) according to claim 8, wherein the transmission means includes a bevel gear (137) arranged at a distal portion of the drive shaft (130).

10. The catheter (100) according to claim 8 or claim 9, wherein there are included a lumen (134) formed by being elongated in the inside of the drive shaft (130) and including an opening portion (135) by which excavated pieces of the occluding object (30) are pulled-in, and excavated-piece suction means (160, 162) communicated with the lumen (122) of the insertion member (120) and the lumen of the drive shaft (130); and
the support portion (142) of the removal mechanism (140) includes a center opening portion (144) communicated with the lumen (138) of the insertion member (120) .

11. The catheter (100) according to any one of claims 8 to 10, wherein when the removal mechanism (140) is compressed onto the occluding object (30) and the drive shaft (130) is rotated, the rotation bodies of the removal mechanism (140) rotate and excavate the occluding object (30), and the excavated object is fed-in inside the lumen (122) of the insertion member (120) and inside the drive shaft (130).

## Patentansprüche

1. Katheter (100), welcher Folgendes umfasst:
einen Hüllenabschnitt (110), welcher ein Lumen (112) aufweist, das sich längs im Inneren davon erstreckt, und welcher in ein röhrenförmiges Organ (20) innerhalb eines lebenden Körpers (10) eingeführt werden kann;
ein Einführungselement (120), welcher ein Lumen (122) aufweist, das sich längs im Inneren davon erstreckt, und welches in Bezug auf das Lumen (112) des Hüllenabschnittes (110) frei verschiebbar und drehbar angeordnet ist; und
einen Entfernungsmechanismus (140) zum Entfernen eines Objektes (30), welches das röhrenförmige Organ (20) innerhalb des lebenden Körpers (10) verstopft, wobei
der Entfernungsmechanismus (140) einen Stützabschnitt (142), der an einem distalen Abschnitt des Einführungselementes (120) angeordnet ist, und mehrere Drehkörper (152A-152C), die an dem Stützabschnitt (142) angeordnet und mit Schneidkanten (154A-154C, 156A-156C) zum Abtragen des verstopfenden Objektes (30) versehen sind, beinhaltet, und
der Stützabschnitt (142) eine Drehachse beinhaltet, die sich in einer Richtung erstreckt, welche die Achse (A) des Einführungselementes (120) schneidet, wobei der Stützabschnitt (142) die mehreren Drehkörper, die mit den Schneidkanten zum Abtragen des verstopfenden Objektes versehen sind, bzw. Punkte, an welchen die verlängerten Linien der Drehachsen der Drehkörper die Achse des Einführungselementes schneiden, unterschiedlich sind, beinhaltet.

2. Katheter (100) nach Anspruch 1, wobei jeder Drehkörper (152A-152C) etwa eine konische Form mit einer Verjüngung in Richtung der distalen Richtung seiner Drehachse aufweist.

3. Katheter (100) nach Anspruch 2, wobei die Schneidkante von jedem Drehkörper zahnradförmig und durch Bildung eines Räderwerkes geformt ist, und das Schneidwerk von jedem Drehkörper (152A-152C) derart angeordnet ist, dass es sich gegenseitig unabhängig in Bezug auf ein Schneidwerk eines benachbarten Drehkörpers dreht.

4. Katheter (100) nach einem der Ansprüche 1 bis 3, welcher ferner Abtragstück-Saugmittel (160, 162) in Verbindung mit dem Lumen (112) des Einführungselementes (120) umfasst, wobei der Entfernungsmechanismus (140) einen mittleren Öffnungsabschnitt (144) in Verbindung mit dem Lumen des Einführungselementes (120) beinhaltet und
die Drehkörper am Umfang des mittleren Öffnungsabschnittes (144) positioniert sind.

5. Katheter (100) nach Anspruch 4, wobei eine Form der Schneidkante von jedem Drehkörper derart ausgebildet ist, dass sie abgetragene Stücke des verstopfenden Objektes (30) zusammen mit der Drehung der Drehkörper in Richtung des mittleren Öffnungsabschnittes (144) nach innen zieht.

6. Katheter (100) nach Anspruch 4 oder 5, welcher ferner Flüssigkeitsablaufmittel (170, 172) für den Ablauf von Flüssigkeit an einem distalen Abschnitt des Hüllenabschnittes (110) umfasst, und
das Flüssigkeitsablaufmittel (170, 172) beinhaltet einen Flusspfad (174), der durch einen Raum ausgebildet ist, welcher zwischen dem Lumen (112) des Hüllenabschnittes (110) und dem äußeren Umfang des Einführungselementes (120) gebildet wird.

7. Katheter (100) nach einem der Ansprüche 1 bis 6, wobei, wenn der Entfernungsmechanismus (140) auf das verstopfende Objekt (30) gedrückt wird, indem das Einführungselement (120) aus dem Hüllenabschnitt (110) vorgeschoben wird, und ferner, wenn das Einführungselement (120) an der Handseite gedreht wird, sich die Drehkörper des Entfernungsmechanismus (140) drehen und das verstopfende Objekt (30) abtragen, und das abgetragene Objekt in das Innere des Lumens (112) des Einführungselementes (120) aufgenommen wird.

8. Katheter (100) nach Anspruch 1, welcher Folgendes umfasst:
eine hohle Antriebswelle (130), die frei drehbar innerhalb des Lumens (112) des Einführungselementes (120) angeordnet ist; und
Übertragungsmittel zum Umwandeln der Drehung der Antriebswelle (130) in die Drehung des Drehkörpers.

9. Katheter (100) nach Anspruch 8, wobei das Übertragungsmittel ein Kegelrad (137) beinhaltet, welches an einem distalen Abschnitt der Antriebswelle (130) angeordnet ist.

10. Katheter (100) nach Anspruch 8 oder 9, wobei Folgendes beinhaltet ist:
ein Lumen (134), das gebildet wird, indem es sich im Inneren der Antriebswelle (130) erstreckt und einen Öffnungsabschnitt (135) beinhaltet, durch welchen abgetragene Stücke des verstopfenden Objektes (30) hineingezogen werden, und Abtragstück-Saugmittel (160, 162) in Verbindung mit dem Lumen (122) des Einführungselementes (120) und dem Lumen der Antriebswelle (130); und
der Stützabschnitt (142) des Entfernungsmechanismus (140) beinhaltet einen mittleren Öffnungsabschnitt (144) in Verbindung mit dem Lumen (138) des Einführungselementes (120).

11. Katheter (100) nach einem der Ansprüche 8 bis 10, wobei, wenn der Entfernungsmechanismus (140) auf das verstopfende Objekt (30) gedrückt wird und die Antriebswelle (130) gedreht wird, sich die Drehkörper des Entfernungsmechanismus (140) drehen und das verstopfende Objekt (30) abtragen, und das abgetragene Objekt wird im Inneren des Lumens (122) des Einführungselementes (120) und im Inneren der Antriebswelle (130) aufgenommen.

## Revendications

1. Cathéter (100) comprenant :
une partie manchon (110) qui comporte un conduit (112) allongé à l'intérieur de celle-ci et qui peut être insérée dans un organe tubulaire (20) à l'intérieur d'un corps vivant (10) ;
un élément d'insertion (120) qui comporte un conduit (122) allongé à l'intérieur de celui-ci et qui est disposé en pouvant coulisser et tourner librement par rapport au conduit (112) de la partie manchon (110) ; et
un mécanisme de retrait (140) pour retirer un objet (30) qui occlut l'organe tubulaire (20) à l'intérieur du corps vivant (10), dans lequel le mécanisme de retrait (140) comprend une partie de support (142) disposée au niveau d'une partie distale de l'élément d'insertion (120) et une pluralité de corps de rotation (152A-152C) disposés au niveau de la partie de support (142) et munis de bords à lame (154A-154C, 156A - 156C) pour excaver l'objet d'occlusion (30), et
la partie de support (142) comprend un axe de rotation qui s'étend dans une direction croisant l'axe (A) de l'élément d'insertion (120), dans lequel la partie de support (142) comporte ladite pluralité de corps de rotation munis desdits bords à lame pour excaver l'objet d'occlusion, et les points au niveau desquels les lignes prolongées des axes de rotation des corps de rotation croisent l'axe de l'élément d'insertion sont différents, respectivement.

2. Cathéter (100) selon la revendication 1, dans lequel chaque corps de rotation (152A-152C) se présente sous une forme approximativement conique ayant une conicité dans la direction distale de son axe de rotation.

3. Cathéter (100) selon la revendication 2, dans lequel le bord à lame de chaque corps de rotation est en forme d'engrenage et est formé en formant un train, et le train de lames de chaque corps de rotation (152A-152C) est disposé de manière à tourner mutuellement de façon indépendante par rapport à un train de lames d'un corps de rotation voisin.

4. Cathéter (100) selon l'une quelconque des revendications 1 à 3, comprenant en outre un moyen d'aspiration de pièce excavée (160, 162) en communication avec le conduit (112) de l'élément d'insertion (120), le mécanisme de retrait (140) comprend une partie d'ouverture centrale (144) en communication avec le conduit de l'élément d'insertion (120), et
les corps de rotation sont disposés au niveau de la circonférence de la partie d'ouverture centrale (144).

5. Cathéter (100) selon la revendication 4, dans lequel une forme du bord à lame de chaque corps de rotation est constituée de manière à attirer les pièces excavées de l'objet d'occlusion (30) vers la partie d'ouverture centrale (144) en même temps que la rotation des corps de rotation.

6. Cathéter (100) selon la revendication 4 ou la revendication 5, comprenant en outre un moyen (170, 172) d'évacuation de liquide pour évacuer le liquide au niveau d'une partie distale de la partie manchon (110), et le moyen (170, 172) d'évacuation de liquide comprend un trajet d'écoulement (174) constitué par un espace qui est formé entre le conduit (112) de la partie manchon (110) et la circonférence extérieure de l'élément d'insertion (120).

7. Cathéter (100) selon l'une quelconque des revendications 1 à 6, dans lequel le mécanisme de retrait (140) est comprimé sur l'objet d'occlusion (30) par l'avancée de l'élément d'insertion (120) hors de la partie manchon (110) et, en faisant tourner l'élément d'insertion (120) côté manche, les corps de rotation du mécanisme de retrait (140) tournent et excavent l'objet d'occlusion (30), et l'objet excavé est amené à l'intérieur du conduit (112) de l'élément d'insertion (120).

8. Cathéter (100) selon la revendication 1, comprenant :
un axe d'entraînement creux (130) disposé en pouvant tourner librement à l'intérieur du conduit (112) de l'élément d'insertion (120) ; et
un moyen de transmission pour convertir la rotation de l'axe d'entraînement (130) en une rotation du corps de rotation.

9. Cathéter (100) selon la revendication 8, dans lequel le moyen de transmission comprend un engrenage conique (137) disposé au niveau d'une partie distale de l'axe d'entraînement (130).

10. Cathéter (100) selon la revendication 8 ou la revendication 9, dans lequel il est prévu un conduit (134) formé en étant allongé à l'intérieur de l'axe d'entraînement (130) et comprenant une partie d'ouverture (135) par laquelle les pièces excavées de l'objet d'occlusion (30) sont entraînées, et un moyen (160, 162) d'aspiration de pièce excavée en communication avec le conduit (122) de l'élément d'insertion (120) et le conduit de l'axe d'entraînement (130) ; et
la partie de support (142) du mécanisme de retrait (140) comprend une partie d'ouverture centrale (144) en communication avec le conduit (138) de l'élément d'insertion (120).

11. Cathéter (100) selon l'une quelconque des revendications 8 à 10, dans lequel, lorsque le mécanisme de retrait (140) est comprimé sur l'objet d'occlusion (30) et l'axe d'entraînement (130) est mis en rotation, les corps de rotation du mécanisme de retrait (140) tournent et excavent l'objet d'occlusion (30), et l'objet excavé est amené à l'intérieur du conduit (122) de l'élément d'insertion (120) et à l'intérieur de l'axe d'entraînement (130).
